# EUROPEAN PATENT APPLICATION

(11) **EP 3 939 494 A1**
(43) Date of publication of application: **19.01.2022**
(21) Application number: 20185605.1
(22) Date of filing: 14.07.2020
(51) Int. Cl.: A61B 5/00, G01R 33/567, A61B 5/055, A61B 5/0456, A61B 5/08

(54) **SYNCHRONISATION SYSTEM**

(71) Applicant: Koninklijke Philips N.V., 5656 AG Eindhoven (NL)
(72) Inventor: SMINK, Jouke, 5656 AE Eindhoven (NL); KOUWENHOVEN, Marc, 5656 AE Eindhoven (NL)
(74) Representative: Philips Intellectual Property & Standards

(57) **Abstract**

A synchronisation system comprising a sensor to detect a current triggering base event. An analysis module with an arithmetic unit is configured to access prior information on intervals between trigger base events and the latency time of the sensor.

An expectation for occurrence of a subsequent trigger base event is computed from the detected current triggering base event, the prior information on delay between trigger base events and the latency time of the sensor. Thus, the analysis module may take the latency time of the sensor into account for estimating (e.g. statistically) or predicting the subsequent trigger base event from the prior information on the intervals between trigger base events.

## Description

### FIELD OF THE INVENTION

The invention pertains to a synchronisation system, notably for a medical imaging apparatus. The synchronisation system operates to trigger image acquisition by the medical imaging apparatus from a triggering base event.

### BACKGROUND OF THE INVENTION

Such a synchronisation system is known from the US-patent application US2020/0022608**.** The known synchronisation system is a system for generating ECG reference data for triggering MR imaging by an MRI device. The known synchronisation system makes use of initial ECG data that are acquired when the patient is outside the bore of the MRI device. When the patient positioning is complete a reference ECG dataset is acquired. In the reference ECG dataset R-tops are identified and compared to R-tops in the initial ECG dataset to account for magneto-hydrodynamic effects in the ECG waveforms in the reference ECG dataset. Image acquisition is triggered on the basis of the identified R-tops.

### SUMMARY OF THE INVENTION

An object of the invention is to provide a synchronisation system that generates a more accurate expectation for occurrence of a subsequent trigger base event.

This object is achieved by the synchronisation system of the invention comprising
- a sensor to detect a current triggering base event,
- an analysis module including an arithmetic unit configured to access
   - prior information on intervals between trigger base events and
   - latency time of the sensor and to
   - compute an expectation for occurrence of a subsequent trigger base event from
   - the detected current triggering base event,
   - the prior information on delay between trigger base events and
   - the latency time of the sensor.

The synchronisation system of the invention comprises a sensor to detect a trigger base event. The trigger base event is formed by an event that represents a relation to an instance at which commencing image acquisition will properly synchronise with dynamics in/of the subject to be imaged. Such an event forms a proper basis for triggering e.g. acquisition of imaging data. The analysis module is configured, notably in software, to receive prior information on intervals between trigger base events. This prior information may have been recorded by the sensor or may have been acquired separately by a different detection method. The analysis module is further configured to receive the sensor's latency time. The latency time is the time-delay between the sensor generating a detection signal representing the trigger base event and the actual time at which the trigger base event occurred. The latency time may be obtained by a calibration procedure in which the dynamics of the sensor is compared with calibration measurement by way of a detector having a very short latency time. The analysis module has an arithmetic module to compute an estimate for a subsequent trigger base event from the prior information on intervals between trigger base events and the latency time. Thus, the analysis module may take the latency time of the sensor into account for estimating or predicting the subsequent trigger base event from the prior information on the intervals between trigger base events. Accordingly, less advanced requirements on the sensor's latency time are required. In particular there is no need to employ a sensor having a very short latency time that may be technically complicated, expensive and susceptible to failure. Since, the next future trigger base event may be accurately predicted, in spite of latency of the detection on the current trigger base event, the next acquisition of imaging data may be accurately triggered. For example, the accurate trigger of the acquisition of imaging data makes efficient use of a next quiescent phase in which there is little or no motion.

These and other aspects of the invention will be further elaborated with reference to the embodiments defined in the dependent Claims.

In a simple example of the synchronisation system, computation of the expectation for occurrence of the subsequent trigger base event is done as the detected current triggering base event's instance plus the delay between trigger base events from the prior information minus the latency time of the sensor. In this way, account is taken of the latency time of the sensor in that a time span of the latency time has elapsed once the sensor generates its detection signal upon the occurrence of the current trigger base event.

Another implementation of the synchronisation system is configured for cardiac triggering of image acquisition. In this implementation the trigger base events are formed by a series of identifiable features in a cardiography signal, for example the R-tops in successive QRS-complexes in the cardiography signal. The prior information on intervals between trigger base events concerns the intervals between previous successive R-tops.

In a further implementation of the synchronisation system, the analysis module is configured to repeatedly receive the prior information on delay between trigger base events. The analysis module may include an arrhythmia rejection function to discard prior information when prior information on delay between trigger base events deviates from a pre-determined reference range. This implementation of the synchronisation system takes account of occurrence of arrhythmia features that may render the prediction of a subsequent trigger base event, such as the next R-top, unreliable.

The pre-determined reference range may be based on a statistical estimate, such as an average or modal interval between preceding successive R-tops, while not exceeding a pre-determined maximum variation of the average interval. That is, intervals between successive R-tops that differ more than the predefined maximum from the current average or modal value are considered outliers due to arrhythmia and are discarded in the computation of the average or modal value. The maximum variation may be determined from previous series of trigger base events of which it has been established that they occur at regular intervals, that may vary slightly.

In a further embodiment of the synchronisation system, the sensor is a sensor arrangement including
- a camera to acquire a series of images of a subject and
- an image processor to derive the prior information on delay between trigger base events from the series of images.

For example, the camera may be arranged so as to monitor an examination zone in which a patient to be examined is positioned e.g. to be imaged. The images of the subject may show temporal variations of the patient's skin from which the patient's heartbeat pattern, i.e. an effective cardiography signal, may be derived. In this way no separate sensor to measure the trigger base events is required. The image acquisition and the image processing for detection of the trigger base events by the sensor arrangement may take an appreciable latency time. This embodiments of the synchronisation system takes that into account to achieve a reliable estimate of the subsequent trigger base event. The images from the camera may also be employed to monitor for other dynamics, e.g. respiratory triggering form chest motion.

Another example of the synchronisation system of the invention includes e.g. a pulse oximetry sensor, a pulse meter, respiratory rate sensor, or a resistance phlesmography sensor, to acquire a data stream representing motion of a subject. A data processor is configured or programmed to derive the prior information on delay between trigger base events from the data stream representing motion of the subject. The present invention enables to employ simple sensors that may have a substantial latency. Even simple consumer-type sensors that may be incorporated in a wrist watch appear to function with sufficient accuracy and reliability although having a long latency that is corrected for. The latency may form an appreciable fraction of the average separation between successive trigger base events (e.g. R-tops), e.g. the latency may be 1/2, 1/3 or 1/4 of the time separation between successive trigger base events. Such a relatively long latency does not affect accurate estimation or prediction of the next future trigger base event so that efficient use can be made of the next future quiescent phase.

The use of e.g. the camera or pulse oximetry sensor, pulse meter, respiratory rate sensor, or a resistance phlesmography sensor avoids the need of complicated vector electrocardiography in triggered cardiac magnetic resonance imaging.

The invention also pertains to a medical imaging apparatus comprising an image acquisition system and provided with the synchronisation system of the invention. The synchronisation system of the invention generates a trigger signal to the image acquisition system. In this way the image acquisition system is activated to start image acquisition at the expected occurrence of the subsequent trigger base event. Thus, the image acquisition is accurately synchronised with motion of the subject to be imaged and the acquired image data have only a low level of motion corruption. For example, the medical imaging apparatus may be a magnetic resonance examination system and the image acquisition system is configured to acquire successive k-space profiles from which magnetic resonance images can be reconstructed. For example the medical imaging apparatus may be a computed-tomography system, or a nuclear medicine (PET or SPECT) imaging apparatus and the image acquisition system is configured to acquire attenuation profiles or emission profiles from which tomographic images can be reconstructed. Also, the medical imaging apparatus may be an x-ray imaging system with an image acquisition system that is configured to acquired x-ray attenuation projection images.

There a several options to determine the latency of the sensor at hand. The latency value may be obtained from the manufacturer's specification. Another option is to use the average latency based on a study in a number of healthy volunteers. Further, another method would be to use a real-time MRI image acquisition of the heart, e.g. a series of short-axis cardio images, to determines systole and diastole and relate that to the images from the camera observed from the patient's skin.

These and other aspects of the invention will be elucidated with reference to the embodiments described hereinafter and with reference to the accompanying drawing wherein

### BRIEF DESCRIPTION OF THE DRAWINGS

Fig. 1 shows a schematic representation of the synchronisation system of the invention,
Fig. 2 shows a diagrammatic representation of the estimation of a next trigger base event as performed by the synchronisation system of the invention and
Fig. 3 shows a schematic representation of a tomographic imaging system incorporating the synchronisation system of the invention.

### DETAILED DESCRIPTION OF THE EMBODIMENTS

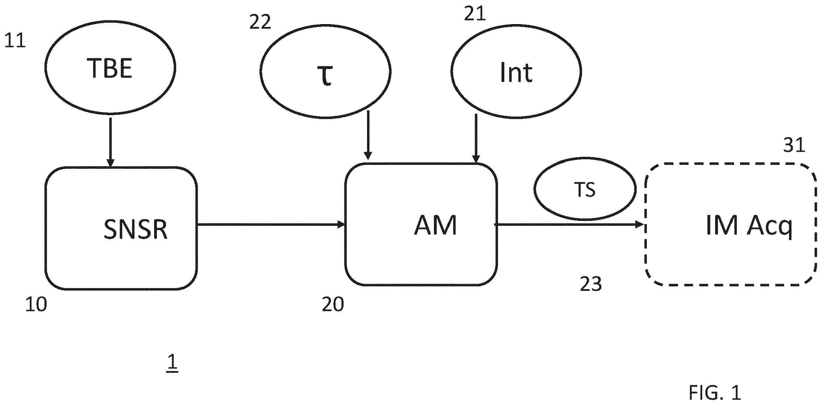

Fig. 1 shows a schematic representation of the synchronisation system 1 of the invention. The synchronisation system functions to estimate a subsequent trigger base event from a detected current trigger base event. The trigger base events are instances that prompt to trigger image acquisition e.g. by a tomographic imaging system. The tomographic imaging system may be a magnetic resonance examination system, a computed tomography system of a nuclear medicine tomographic imaging system. The acquired imaging information may be k-space profiles, attenuation profiles or detected gamma (γ) photons. A magnetic resonance image may be reconstructed form the k-space profiles, a computed tomography image may be reconstructed from (x-ray) attenuation profiles at various orientations. From the detected γ-photons a nuclear medicine tomographic image may be reconstructed. The imaging information in the form of k-space profiles , attenuation profiles or detected photons may be acquired in multiple sets of imaging data at successive intervals, notably during successive equal or comparable motion states of the subject to be imaged (patient to be examined). For example imaging data sets may be acquired an equal, comparable of corresponding cardiac or respiratory phases. The synchronisation system is provided with a sensor 10 to detect a current trigger base event. The current trigger base event may be one of a series or repeating or cyclic trigger bases events that may occur with some degree or even a high degree of temporal regularity. The synchronisation system has an analysis module that has access to timing 21 of previous trigger base events. From the instants of the previous trigger base events the analysis module computes e.g. from a regularity pattern of the occurrences of the previous trigger bases events an estimate for the subsequent next trigger base to occur. Notably, from the timing a previous trigger base events, an representative value of the intervals between successive trigger base events may be derived, e.g. as a statistical estimate representing variations of the regularity of the temporal pattern such as an average or a modal value of the intervals between successive trigger base events. This recognition of regularity in the pattern may be done by way of a neural network. Further, the analysis module 20 has access to the latency time 22 of the sensor 10. This latency represents the time that has elapsed between the actual occurrence of the trigger base event and the sensor outputting a detection signal that represents the occurred trigger base event. The latency time may e.g. be the time between the occurrence of an R-top in an electrocardiogram and the sensor having supplied its output signal in response to the detection of the R-top . The analysis module computes the expected subsequent trigger base events from the instant of the current trigger base event as the representative value of the intervals between trigger base events minus the latency time of the sensor. In other words, the analysis module in fact computes the expected next trigger base event as from the instant of the current trigger base having been detected, rather than from its actual occurrence and take account of the fact that detection is delayed by the latency time. Because the analysis module takes account of the latency time, the invention obviates the need to limit to sensors having a very short latency time; such sensors are often rather expensive. That is, taking account of the sensor's latency time in the estimate of the subsequent trigger even allows to employ relatively inexpensive sensors, or to use sensors that are designed for a different purpose. The invention makes use of a sub-system that is already provide for another function than the triggering. For example, the camera that is provided for monitoring the patient to be examined may also generate the trigger signal. The resulting trigger signal 23 for the subsequent trigger base event is applied to an image acquisition control 31 of a tomographic imaging system. Accordingly, the triggering of the image acquisition control ensures that respective imaging data sets are acquired at equal timings from trigger base events so that the imaging data sets are generated from equal or corresponding (e.g. cardiac or respiratory) motion phases. From these acquired imaging data

sets tomographic images having at most a few and only minor motion artefacts can be reconstructed.

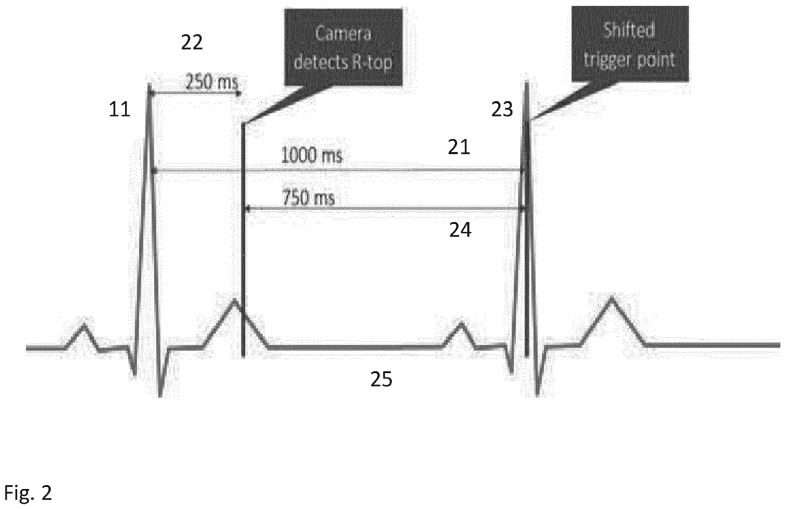

Fig. 2 shows a diagrammatic representation of the estimation of a next trigger base event as performed by the synchronisation system of the invention. By way of example, Fig. 2 shows a part of an electrocardiogram showing successive R-tops 11. Each of the R-top forms a trigger base event. A quiescent heart phase occurs between the successive R-tops in which there is relatively little motion of the anatomy in the region-of-interest of the patient to be examined. Fig. 2 shows two R-tops 11 of a part of the electrocardiogram with in a series of R-tops. Between the R-tops there occurs a diastolic heart phase 25. In this quiescent heart phase in which there is only little motion of the heart and surrounding tissue. Imaging data, such a k-space profiles or attenuation profiles acquired in the quiescent heart phase are at most little corrupted by motion. In the example electrocardiogram of Fig. 2, the successive R-tops 11 are 1000ms apart. The earlier R-top is detected by the sensor, which outputs its detection signal with a delay of 250ms, its latency time. From the time separation of earlier R-tops (not shown) the expected separation between successive R-tops may be estimated, in the present example about 1000ms. Accordingly, from the detection of the earlier R-top about 250ms from its occurrence, the next R-top is estimated to be expected at about 750ms from the detection of the R-top. Accordingly, it may be derived that the remainder of the current quiescent phase lasts for about 750ms and the next quiescent phase may be triggered about 1000ms from te current R-top being detected. This enables accurate triggering of the image acquisition control 31 so as to acquired imaging data that are (almost) not corrupted by motion. Thus, the diagnostic quality of the reconstructed magnetic resonance image is improved

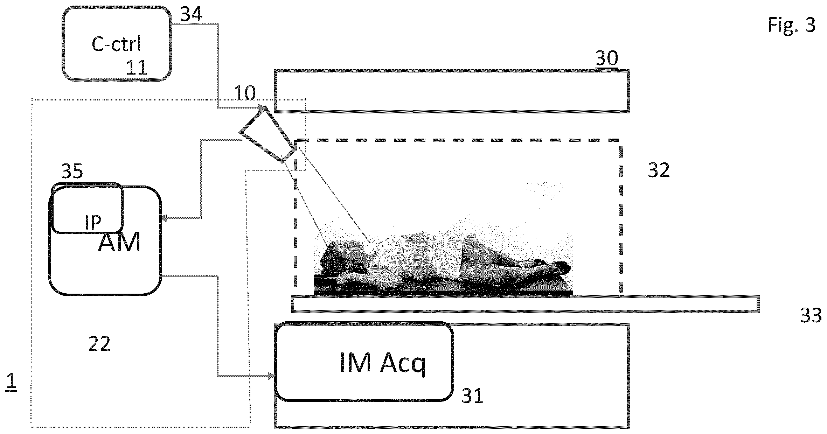

Fig. 3 shows a schematic representation of a tomographic imaging system 30 incorporating the synchronisation system 1 of the invention. The tomographic imaging system may be a magnetic resonance examination system having an examination zone 32 in the bore of the magnetic resonance examination system's magnet. Another example of the tomographic imaging system is a computed-tomography (CT) of a nuclear medicine (NM) imaging system having the examination zone in the CT-system's gantry. A patient carrier 33 supports a patient to be examined placed in the examination zone 32. The camera 10 is mounted to the imaging system's structure e.g. the gantry of magnet support and has its field-of-view in the examination zone, notably so as to acquire a series of images from the region-of-interest of the patient to be examined, e.g. from the patient's thorax or face. The series of images may be represented by streaming video data. The camera is controlled by a camera control system 34 to control the operation of the camera. The output video data stream of the camera is applied to the analysis module 22. The output data stream of the camera may represent motion of the patient's thorax or temporal variation of the skin colour of the patient's face. From the periodicity of the motion or the skin colour variations, electrocardiogram data, notably the R-tops, may be derived.

The analysis module 22 contains an image processing unit that identifies the occurrence of trigger base events such as R-tops in the electrocardiogram from the output data stream, i.e. from the series of images acquired by the camera 10. From the identified R-tops, the analysis module computes the expected time separation between one R-top and the next, notably, between the currently detected R-top and the expected next future R-top. This may be done on the basis of averaging over a sliding time window of a number of previous R-tops. In a more sophisticated implementation the expected time separation is determined on the basis of a higher order polynomial fitting to previously detected R-tops or even by way of artificial intelligence. The analysis module generates the an expectation 24 for occurrence of a subsequent trigger base event, next future R-top and applies the expectation 24 as a trigger signal to the image acquisition control 31 of the tomographic imaging system. In response to receipt of the trigger signal, the image acquisition control 31 drives the image acquisition function of the tomographic imaging system to acquire imaging data during the current quiescent phase of the patient's heart. For a magnetic resonance examination system the image acquisition control drives the magnetic resonance examination system's radio frequency system to generated excitation RF pulses and to receive magnetic resonance signals (k-space profiles) in response to the excitation. For a computed-tomography system the image acquisition system drives the computed tomography system's x-ray source and x-ray detector to generate attenuation profiles. For a nuclear medicine imaging system, the imaging acquisition control controls the nuclear medicine imaging system's detection system to acquire γ-radiation.

## Claims

1. A synchronisation system comprising
- a sensor (10) to detect a current triggering base event (11),
- an analysis module (20) including an arithmetic unit configured to access
- prior information (21) on intervals between trigger base events and
- latency time (22) of the sensor and to
- compute an expectation (24) for occurrence of a subsequent trigger base event (23) from
- the detected current triggering base event,
- the prior information on delay between trigger base events and
- the latency time of the sensor.

2. A synchronisation system as claimed in Claim 1, wherein the arithmetic unit is configured to compute an expectation for occurrence of the subsequent trigger base event as the detected current triggering base event's instance plus the prior information on delay between trigger base events minus the latency time of the sensor.

3. A synchronisation system as claimed in any one of Claims 1 or 2, wherein the triggering base events are R-tops of an electocardiographical dataset and the prior information on delay between trigger base events is a statistical estimate such as an average or modal interval between successive R-tops.

4. A synchronisation system as claimed in any one of the preceding Claims, wherein the analysis module is configured to repeatedly receive the prior information on delay between trigger base events and the analysis module includes an arrhythmia rejection function to discard prior information when prior information on delay between trigger base events deviates from a pre-determined reference range.

5. A synchronisation system as claimed in Claim 4, wherein the pre-determined reference is based on an average or modal interval between preceding successive R-tops, accounting for a pre-determined maximum variation of the average interval.

6. A synchronisation system as claimed in any one of the preceding Claims, wherein the sensor is formed by a sensor arrangement including
- a camera to acquire a series of images of a subject and
- an image processor to derive the prior information on delay between trigger base events from the series of images.

7. A synchronisation system as claimed in any one of the preceding Claims, wherein the sensor is formed by a sensor arrangement including
- a pulse oximetry sensor, a pulse meter, respiratory rate sensor, or a resistance phlesmography sensor, to acquire a data stream representing motion of a subject and
- an data processor to derive the prior information on delay between trigger base events from the data stream representing motion of the subject.

8. A medical imaging apparatus including an image acquisition system and a synchronisation system as claimed in any one of Claims 1 to 7 to trigger image acquisition.
